# EUROPEAN PATENT SPECIFICATION

(11) **EP 4 329 041 B1**
(45) Date of publication and mention of the grant of the patent: **05.08.2026**
(21) Application number: 22911662.9
(22) Date of filing: 29.11.2022
(51) Int. Cl.: H01M 10/0567, H01M 10/052, H01M 4/525, H01M 10/0525, H01M 10/0568, H01M 10/0569, H01M 4/02

(54) **NON-AQUEOUS ELECTROLYTE INCLUDING ADDITIVE FOR NON-AQUEOUS ELECTROLYTE, AND LITHIUM SECONDARY BATTERY INCLUDING THE SAME**
NICHTWÄSSRIGER ELEKTROLYT UMFASSEND ADDITIV FÜR NICHTWÄSSRIGEN ELEKTROLYT UND LITHIUMSEKUNDÄRBATTERIE DAMIT
ÉLECTROLYTE NON AQUEUX COMPORTANT UN ADDITIF POUR ÉLECTROLYTE NON AQUEUX, ET BATTERIE SECONDAIRE AU LITHIUM LE COMPORTANT

(30) Priority: 21.12.2021 KR 20210183693
(43) Date of publication of application: 28.02.2024
(73) Proprietor: LG Energy Solution, Ltd., Seoul 07335 (KR)
(72) Inventor: PARK, Sung Guk, Daejeon 34122 (KR); OH, Jeong Woo, Daejeon 34122 (KR); LEE, Chul Haeng, Daejeon 34122 (KR)
(74) Representative: Hoffmann Eitle
(86) International application number: PCT/KR2022/019062
(87) International publication number: WO 2023/121028

(56) References cited:
- CN-A- 101 345 325
- CN-A- 103 000 942
- CN-A- 110 707 360
- CN-A- 110 707 360
- KR-A- 20210 064 175
- KR-A- 20210 064 175
- US-A1- 2012 171 576

## Description

### TECHNICAL FIELD

### Cross-reference to Related Applications

This application claims the benefit of Korean Patent Application No. 10-2021-0183693, filed on December 21, 2021.

### Technical Field

The present invention relates to a non-aqueous electrolyte including an additive for a non-aqueous electrolyte, and a lithium secondary battery including the same.

### BACKGROUND ART

Recently, as application fields of a lithium secondary battery have rapidly expanded to not only the power supply of electronic devices such as electricity, electronics, communications, and computers but also the power storage supply of large-area devices such as automobiles and power storage devices, a demand for a secondary battery having high capacity, high output, and high stability has been increasing.

In particular, in a lithium secondary battery for automobiles, high capacity, high output, and long-term service life characteristics has been becoming important. In order to increase the capacity of the secondary battery, a high-nickel positive electrode active material having high energy density but low stability can be used, or the secondary battery can be driven with a high voltage.

However, when the secondary battery is driven under the above conditions, as charging and discharging proceeds, the surface structure of an electrode or a film formed on the surface of positive/negative electrode deteriorates due to a side reaction caused by the deterioration of an electrolyte, and thus transition metal ions may be eluted from the surface of the positive electrode. As described above, since the eluted transition metal ions are electro-deposited on the negative electrode and reduce passivation ability of a solid electrolyte interphase (SEI), there occurs a limitation in that the negative electrode deteriorates.

This deterioration phenomenon of the secondary battery tends to be further accelerated when the potential of the positive electrode is increased or when the battery is exposed to high temperatures.

In addition, when the lithium secondary battery is continuously used for a long period of time or left to stand at high temperatures, gas is generated, thereby causing a so-called swelling phenomenon in which the thickness of the battery increases, and it is known that the amount of gas generated in this case depends on the state of the SEI.

Therefore, in order to solve such problems, research and development on methods capable of suppressing the elution of metal ions from the positive electrode and forming a stable SEI film on the negative electrode, thereby reducing the swelling phenomenon of the secondary battery and increasing the stability at high temperatures have been attempted.
CN 110707360 A relates to a lithium ion battery electrolyte which contains an imidazole covalent compound which accounts for 0.1%-1% of the total weight of the electrolyte and has a substituent with unsaturated olefinic bonds.
KR 10-2021-0064175 A relates to a non-aqueous electrolyte for a lithium secondary battery including a fluorine-containing compound capable of forming a stable film on the surface of an electrode as an additive, and a lithium secondary battery including the same.

### DISCLOSURE OF THE INVENTION

### TECHNICAL PROBLEM

An aspect of the present invention provides an additive for a non-aqueous electrolyte capable of suppressing the degradation of a positive electrode, reducing side reactions between a positive electrode and an electrolyte, and forming a stable SEI film on a negative electrode.

Another aspect of present invention provides a non-aqueous electrolyte having improved stability at high temperatures by including the additive for a non-aqueous electrolyte.

Another aspect of the present invention provides a lithium secondary battery having improved overall performance by including the non-aqueous electrolyte, thereby having improved high-temperature cycle characteristics and high-temperature storage characteristics.

### TECHNICAL SOLUTION

According to an aspect of the present invention, there is provided a non-aqueous electrolyte containing a lithium salt including LiPF₆, an organic solvent, and an additive for a non-aqueous electrolyte represented by Formula 1 below, with the additive for a non-aqueous electrolyte being contained in an amount of 0.1 parts by weight to 5 parts by weight based on 100 parts by weight of the non-aqueous electrolyte:

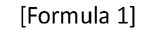

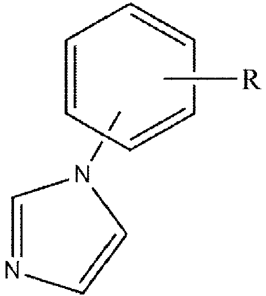

In Formula 1 above, R is any one selected from the group consisting of a halogen element and a haloalkyl group having 1 to 10 carbon atoms.

According to another aspect of the present invention, there is provided a lithium secondary battery including the non-aqueous electrolyte.

### ADVANTAGEOUS EFFECTS

The compound represented by Formula 1 above provided as the additive for a non-aqueous electrolyte according to the present invention is a compound in which a halogen element or a haloalkyl group is substituted together with an imidazole group at a benzene ring, and a stable solid electrolyte interphase (SEI) film may be formed on the surface of the negative electrode while minimizing an increase in resistance of the lithium secondary battery. Therefore, it is possible to suppress the degradation in passivation ability of the SEI at high temperatures, thereby preventing the negative electrode from deteriorating.

In addition, PF₅, which is a charge and discharge by-product of LiPF₆ used as a lithium salt, and HF, which is generated by an additional reaction of moisture and PF₅, attack the positive electrode, the negative electrode, and the film components of the battery, thereby deteriorating life characteristics. Specifically, in the positive electrode, the transition metal is eluted by HF, and the eluted transition metal is re-deposited, thereby increasing the resistance of the positive electrode. In the negative electrode, HF is electrodeposited, thereby causing various limitations such as the self-discharge of the negative electrode, the destruction of the SEI film, the consumption of additional lithium ions due to SEI regeneration, an increase in resistance of the secondary battery, and gas generation. Since the compound represented by Formula 1 above provided as the additive for a non-aqueous electrolyte according to the present invention has a high binding energy with PF₅ generating HF, there is an effect of suppressing the decomposition reaction of PF₅ to increase the durability of the battery.

Therefore, when the non-aqueous electrolyte of the present invention including the compound of Formula 1 above is used, the electrode-electrolyte interface, which is stable even at a high temperature and has a low resistance, may be formed, and thus a lithium secondary battery having improved high-temperature cycle characteristics and high-temperature storage characteristics and improved overall performance may be achieved.

### MODE FOR CARRYING OUT THE INVENTION

It will be understood that words or terms used in the specification and claims shall not be interpreted as the meaning defined in commonly used dictionaries, and it will be further understood that the words or terms should be interpreted as having a meaning that is consistent with their meaning in the context of the relevant art and the technical idea of the invention, based on the principle that an inventor may properly define the meaning of the words or terms to best explain the invention.

It will be further understood that the terms "include," "comprise," or "have" in this specification specify the presence of stated features, numbers, steps, elements, or combinations thereof, but do not preclude the presence or addition of one or more other features, numbers, steps, elements, or combinations thereof.

Also, the expressions "a" and "b" in the description of "a to b carbon atoms" in the specification each denote the number of carbon atoms included in a specific functional group. That is, the functional group may include "a" to "b" carbon atoms. For example, the expression "alkylene group having 1 to 5 carbon atoms" denotes an alkylene group including 1 to 5 carbon atoms, that is, -CH₂-, -CH₂CH₂-, -CH₂CH₂CH₂-, -CH₂(CH₃)CH-,-CH(CH₃)CH₂-, and -CH(CH₃)CH₂CH₂-.

Furthermore, in the present specification, the expression "alkylene group" denotes a branched or unbranched divalent saturated hydrocarbon group.

In addition, in the present specification, an alkyl group or an alkylene group may all be substituted or unsubstituted. Unless otherwise defined, the term "substituted" means that at least one hydrogen bonded to carbon is substituted with an element other than hydrogen, and for example, it means being substituted with an alkyl group having 1 to 20 carbon atoms, an alkenyl group having 2 to 20 carbon atoms, an alkynyl group having 2 to 20 carbon atoms, an alkoxy group having 1 to 20 carbon atoms, a cycloalkyl group having 3 to 12 carbon atoms, a cycloalkenyl group having 3 to 12 carbon atoms, a heterocycloalkyl group having 3 to 12 carbon atoms, a heterocycloalkenyl group having 3 to 12 carbon atoms, an aryloxy group having 6 to 12 carbon atoms, a halogen atom, a fluoroalkyl group having 1 to 20 carbon atoms, a nitro group, an aryl group having 6 to 20 carbon atoms, a heteroaryl group having 2 to 20 carbon atoms, a haloaryl group having 6 to 20 carbon atoms, *etc.*

Hereinafter, the present invention will be described in more detail.

### Non-aqueous Electrolyte

The non-aqueous electrolyte according to the present invention includes a compound represented by Formula 1 below as an additive. A secondary battery including the non-aqueous electrolyte using the additive compound of the present invention may have excellent high-temperature cycle characteristics and high-temperature storage characteristics since deterioration by caused by interfacial reactions at high temperatures is suppressed.

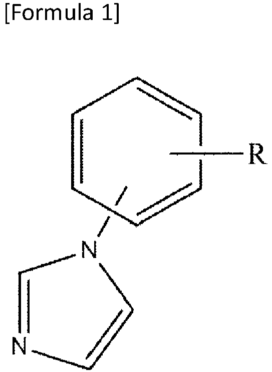

The compound of Formula 1 above may minimize an increase in resistance of the lithium secondary battery by including an imidazole structure, thereby forming a stable solid electrolyte interphase (SEI) film on the surface of the negative electrode. Therefore, it is possible to suppress the degradation in passivation ability of the SEI at high temperatures, thereby preventing the negative electrode from deteriorating. In particular, the compound of Formula 1 has an excellent effect of reducing gas because H of a nitrogen atom in the imidazole is substituted with a benzene ring, and thus the amount of hydrogen generated is less than the case where H is present in nitrogen.

In addition, the compound of Formula 1 above may form a passivation layer capable of securing excellent oxidation resistance by a halogen element having excellent flame retardancy and nonflammability included in the molecular structure. As a result, side reactions of the electrodes and the electrolyte solution are controlled, and the lithium secondary battery having improved life characteristics may be provided. Like the compound of Formula 1, when halogen is not directly substituted at the imidazole group and a halogen element is substituted via a benzene ring, F may easily leave from the benzene structure, thereby further facilitating film formation.

In Formula 1 above, R is any one selected from the group consisting of a halogen element and a haloalkyl group having 1 to 10 carbon atoms. The haloalkyl group means an alkyl group substituted with at least one halogen element. Specifically, R may be any one selected from the group consisting of F, Cl, and a haloalkyl group having 1 to 5 carbon atoms, preferably, any one selected from the group consisting of F, CF₃, and CF₂CF₃, and most preferably F. When F is directly linked to the benzene ring, F leaves well to participate in the formation of SEI, and thus effects of reducing a high-temperature gas and maintaining capacity may be better.

Preferably, the compound of Formula 1 above may be a compound represented by Formula 1-1 below or a compound represented by Formula 1-2 below:

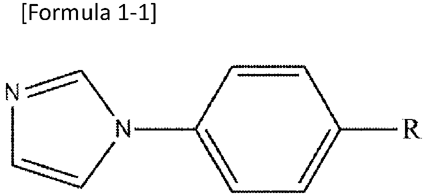

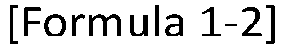

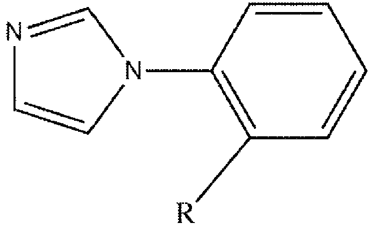

In Formulae 1-1 and 1-2 above, R may be any one selected from the group consisting of a halogen element and a haloalkyl group having 1 to 10 carbon atoms. Specifically, R may be any one selected from the group consisting of F, Cl, and a haloalkyl group having 1 to 5 carbon atoms, preferably, any one selected from the group consisting of F, CF₃, and CF₂CF₃, and most preferably F. When F is directly linked to the benzene ring, F leaves well to participate in the formation of SEI, and thus effects of reducing a high-temperature gas and maintaining capacity may be better.

The additive for a non-aqueous electrolyte according to the present invention may be any one among the compounds represented by 2-1 to 2-4 below:

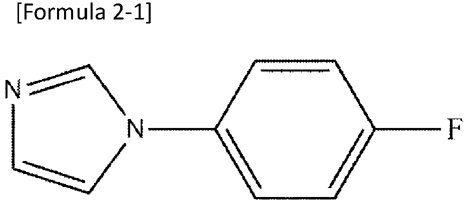

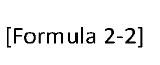

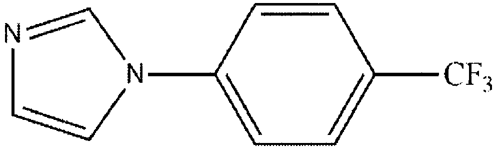

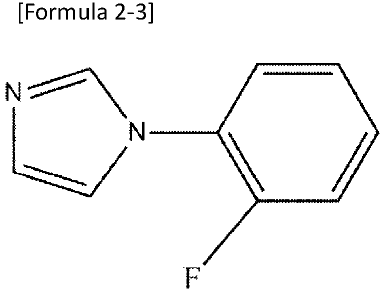

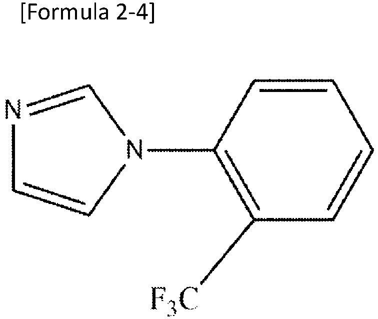

The additive for a non-aqueous electrolyte according to the present invention is included in an amount of 0.1 parts by weight to 5 parts by weight. Preferably the additive for a non-aqueous electrolyte according to the present invention may be included in an amount of 0.1 parts by weight to 1 parts by weight, and more preferably, 0.1 parts by weight to 0.5 parts by weight based on 100 parts by weight of the non-aqueous electrolyte. If the content of the compound represented by Formula 1 above satisfies the above range, the effect of forming a film on the negative electrode is sufficient, thereby inhibiting the transition metal, which is eluted from the positive electrode active material, from being adsorbed on the negative electrode, and the viscosity of the electrolyte is maintained in an appropriate level, and thus there is an effect of having excellent rate capability or life characteristics during storing at high temperatures.

The non-aqueous electrolyte according to the present invention may contain a lithium salt, an organic solvent, or other electrolyte additives.

The non-aqueous electrolyte according to the present invention may contain LiPF₆ as a lithium salt in terms of excellent high-temperature stability. In this case, the compound represented by Formula 1 has a high binding energy with PF₅ which is a charge and discharge by-product of LiPF₆ used as a lithium salt, and thus may have an effect of increasing the durability of the battery by suppressing the additional decomposition reaction of PF₅.

In addition, the non-aqueous electrolyte of the present invention may include, as a lithium salt other than LiPF₆ above, a lithium salt selected from the group consisting of LiCl, LiBr, Lil, LiBF₄, LiClO₄, LiB₁₀Cl₁₀, LiAlCl₄, LiAlO₂, LiSO₃CH₃, LiSO₃CF₃, LiCO₂CH₃, LiCO₂CF₃, LiAsF₆, LiSbF₆, LiN(SO₂F)₂ (lithium bis(fluorosulfonyl)imide, LiFSI), LiN(SO₂CF₂CF₃)₂ (lithium bis(perfluoroethanesulfonyl)imide, LiBETI), and LiN(SO₂CF₃)₂ (lithium bis(trifluoromethanesulfonyl)imide, LiTFSI). In addition to them, a lithium salt typically used in an electrolyte of a lithium secondary battery may be used without limitation.

The lithium salt included in the non-aqueous electrolyte of the present invention may be appropriately changed in a normally usable range, but may be included in a concentration of 0.5 M to 4.0 M, preferably, 0.5 M to 3.0 M, and more preferably, 0.8 M to 2.0 M in the electrolyte in order to obtain an optimum effect of forming a film for preventing corrosion on the surface of an electrode. When the concentration of the lithium salt satisfies the above range, there is a sufficient effect of improving cycle characteristics during high-temperature storage of a lithium secondary battery, and the viscosity of the non-aqueous electrolyte is suitable, so that the impregnability of the electrolyte may be improved.

The organic solvent may include at least one organic solvent selected from the group consisting of a cyclic carbonate-based organic solvent, a linear carbonate-based organic solvent, a linear ester-based organic solvent, and a cyclic ester-based organic solvent.

Specifically, the organic solvent may include a cyclic carbonate-based organic solvent, a linear carbonate-based organic solvent, and a mixed organic solvent thereof.

The cyclic carbonate-based organic solvent is an organic solvent which may well dissociate the lithium salt in the electrolyte due to high permittivity as a highly viscous organic solvent, wherein specific examples of the cyclic carbonate-based organic solvent may be at least one organic solvent selected from the group consisting of ethylene carbonate (EC), propylene carbonate (PC), 1,2-butylene carbonate, 2,3-butylene carbonate, 1,2-pentylene carbonate, 2,3-pentylene carbonate, and vinylene carbonate, and, among them, the cyclic carbonate-based organic solvent may include ethylene carbonate.

Also, the linear carbonate-based organic solvent is an organic solvent having low viscosity and low permittivity, wherein typical examples of the linear carbonate-based organic solvent may be at least one organic solvent selected from the group consisting of dimethyl carbonate (DMC), diethyl carbonate (DEC), dipropyl carbonate, ethyl methyl carbonate (EMC), methylpropyl carbonate, and ethylpropyl carbonate, and the linear carbonate-based organic solvent may specifically include ethyl methyl carbonate (EMC).

Furthermore, the organic solvent may further include at least one ester-based organic solvent selected from the group consisting of a linear ester-based organic solvent and a cyclic ester-based organic solvent in addition to at least one carbonate-based organic solvent selected from the group consisting of the cyclic carbonate-based organic solvent and the linear carbonate-based organic solvent, in order to prepare an electrolyte having high ionic conductivity.

Specific examples of the linear ester-based organic solvent may be at least one organic solvent selected from the group consisting of methyl acetate, ethyl acetate, propyl acetate, methyl propionate, ethyl propionate, propyl propionate, and butyl propionate.

Also, the cyclic ester-based organic solvent may include at least one organic solvent selected from the group consisting of γ-butyrolactone, γ-valerolactone, γ-caprolactone, σ-valerolactone, and ε-caprolactone.

Meanwhile, if necessary, any organic solvent commonly used in a non-aqueous electrolyte may be additionally used without limitation as the organic solvent. For example, at least one organic solvent among an ether-based organic solvent, a glyme-based organic solvent, and a nitrile-based organic solvent may be further included.

As the ether-based solvent, any one selected from the group consisting of dimethyl ether, diethyl ether, dipropyl ether, methyl ethyl ether, methyl propyl ether, ethyl propyl ether, 1,3-dioxolane (DOL), and 2,2-bis(trifluoromethyl)-1,3- dioxolane (TFDOL) or a mixture of two or more thereof may be used, but the ether-based solvent is not limited thereto.

The glyme-based organic solvent is a solvent having higher dielectric constant and lower surface tension than the linear carbonate-based organic solvent and having lower reactivity with metal, wherein the glyme organic solvent may include at least one selected from the group consisting of dimethoxyethane (glyme, DME), diglyme, triglyme, and tetraglyme (TEGDME), but the glyme organic solvent is not limited thereto.

The nitrile-based organic solvent may include at least one selected from the group consisting of acetonitrile, propionitrile, butyronitrile, valeronitrile, caprylonitrile, heptanenitrile, cyclopentane carbonitrile, cyclohexane carbonitrile, 2-fluorobenzonitrile, 4-fluorobenzonitrile, difluorobenzonitrile, trifluorobenzonitrile, phenylacetonitrile, 2-fluorophenylacetonitrile, and 4-fluorophenylacetonitrile, but the nitrile organic solvent is not limited thereto.

In addition, the non-aqueous electrolyte of the present invention may further include, if necessary, an electrolyte additive known in the art in the non-aqueous electrolyte in order to prevent the non-aqueous electrolyte from being decomposed in a high-output environment and causing a negative electrode to collapse, or to further improve low-temperature high-rate discharge characteristics, high-temperature stability, overcharge prevention, an effect of suppressing battery expansion at high temperatures, and the like.

Representative examples of the additional electrolyte additive may include at least one additive for forming an SEI film selected from the group consisting of a cyclic carbonate-based compound, a halogen-substituted carbonate-based compound, a sultone-based compound, a sulfate-based compound, a phosphate-based compound, a borate-based compound, a nitrile-based compound, a benzene-based compound, an amine-based compound, a silane-based compound, and a lithium salt-based compound.

The cyclic carbonate-based compound may include vinylene carbonate (VC) or vinyl ethylene carbonate.

The halogen-substituted carbonate-based compound may include fluoroethylene carbonate (FEC).

The sultone-based compound may include at least one compound selected from the group consisting of 1,3-propane sultone (PS), 1,4-butane sultone, ethane sultone, 1,3-propene sultone (PRS), 1,4-butene sultone, and 1-methyl-1,3-propene sultone.

The sulfate-based compound may include ethylene sulfate (Esa), trimethylene sulfate (TMS), or methyl trimethylene sulfate (MTMS).

The phosphate-based compound may include at least one compound selected from the group consisting of lithium difluorobis(oxalato)phosphate, lithium difluorophosphate, tetramethyl trimethylsilyl phosphate, trimethylsilyl phosphite, tris(2,2,2-trifluoroethyl)phosphate, and tris(trifluoroethyl)phosphite.

The borate-based compound may include tetraphenylborate, lithium oxalyldifluoroborate (LiODFB), and lithium bis(oxalato)borate (LiB(C₂O₄)₂, LiBOB).

The nitrile-based compound may include at least one compound selected from the group consisting of succinonitrile, adiponitrile, acetonitrile, propionitrile, butyronitrile, valeronitrile, caprylonitrile, heptanenitrile, cyclopentanecarbonitrile, cyclohexanecarbonitrile, 2-fluorobenzonitrile, 4-fluorobenzonitrile, difluorobenzonitrile, trifluorobenzonitrile, phenylacetonitrile, 2-fluorophenylacetonitrile, and 4-fluorophenylacetonitrile.

The benzene-based compound may include fluorobenzene, the amine-based compound may include triethanolamine or ethylenediamine, and the silane-based compound may include tetravinylsilane.

The lithium salt-based compound is a compound different from the lithium salt included in the non-aqueous electrolyte, and may be lithium difluorophosphate (LiDFP), LiPO₂F₂, LiBF₄, or the like.

Among the additional electrolyte additives, when a combination of vinylene carbonate (VC), 1,3-propane sultone (PS), and ethylene sulfate (Esa) is further included, it is possible to form a more robust SEI film on the surface of a negative electrode during an initial activation process of a secondary battery, and to suppress the generation of a gas which may be generated due to the decomposition of an electrolyte at high temperatures, thereby improving high-temperature stability of the secondary battery.

Meanwhile, the additional additives may be used as a mixture of two or more thereof, and may be included in an amount of 0.1 wt% to 10 wt%, particularly 0.2 wt% to 8 wt%, and preferably 0.5 wt% to 8 wt% based on the total weight of the non-aqueous electrolyte. When the content of the additional electrolyte additives satisfies the above range, there is a more excellent effect of improving ionic conductivity and cycle characteristics.

### Lithium Secondary Battery

The present invention also provides a lithium secondary battery including the non-aqueous electrolyte.

Specifically, the lithium secondary battery includes a positive electrode including a positive electrode active material, a negative electrode including a negative electrode active material, a separator disposed between the positive electrode and the negative electrode, and the above-described non-aqueous electrolyte.

In this case, the lithium secondary battery of the present invention may be prepared according to a typical method known in the art. For example, after an electrode assembly is formed by sequentially stacking a positive electrode, a negative electrode, and a separator disposed between the positive electrode and the negative electrode, the lithium secondary battery of the present invention may be prepared by inserting the electrode assembly into a battery case, and injecting the non-aqueous electrolyte according to the present invention.

### (1) Positive Electrode

The positive electrode may be prepared by coating a positive electrode collector with a positive electrode material mixture slurry including a positive electrode active material, a binder, a conductive agent, and a solvent.

The positive electrode collector is not particularly limited so long as it has conductivity without causing adverse chemical changes in the battery, and, for example, stainless steel, aluminum, nickel, titanium, fired carbon, or aluminum or stainless steel that is surface-treated with one of carbon, nickel, titanium, silver, or the like may be used.

The positive electrode active material is a compound capable of reversibly intercalating and deintercalating lithium, wherein the positive electrode active material may specifically include a lithium composite metal oxide including lithium and at least one metal such as cobalt, manganese, nickel, or aluminum. More specifically, the lithium metal oxide may include a lithium-manganese-based oxide (e.g., LiMnO₂, LiMn₂O₄, etc.), a lithium-cobalt-based oxide (e.g., LiCoO₂, etc.), a lithium-nickel-based oxide (e.g., LiNiO₂, etc.), a lithium-nickel-manganese-based oxide (e.g., LiNi_{1-Y}Mn_{Y}O₂ (where 0<Y<1), LiMn_{2-z}Ni_{z}O₄ (where 0<Z<2), etc.), a lithium-nickel-cobalt-based oxide (e.g., LiNi_{1-Y1}Co_{Y1}O₂ (where 0<Y1<1), etc.), a lithium-manganese-cobalt-based oxide (e.g., LiCo_{1-Y2}Mn_{Y2}O₂ (where 0<Y2<1), LiMn_{2-z1}Co_{z1}O₄(where 0<Z1<2), etc.), a lithium-nickel-manganese-cobalt-based oxide (e.g., Li(NiₚCo_{q}Mn ᵣ)O₂ (where 0<p<1, 0<q<1, 0< r<1, and p+q+r=1) or Li(Niₚ₁Co_{q1}Mnᵣ₁)O₄ (where 0<p1<2, 0<q1<2, 0<r1<2, and p1+q1+r1=2), etc.), or a lithium-nickel-cobalt-transition metal (M) oxide (e.g., Li(Niₚ₂Co_{q2}Mnᵣ₂Mₛ₂)O₂ (where M is selected from the group consisting of Al, Fe, V, Cr, Ti, Ta, Mg, and Mo, and p2, q2, r2, and s2 are atomic fractions of each independent elements, wherein 0<p2<1, 0<q2<1, 0<r2<1, 0<S2<1, and p2+q2+r2+S2=1), etc.), and any one thereof or a compound of two or more thereof may be included.

Among these materials, in terms of the improvement of capacity characteristics and stability of the battery, the lithium metal oxide may include LiCoO₂, LiMnO₂, LiNiO₂, a lithium nickel manganese cobalt oxide (e.g., Li(Ni_{1/3}Mn_{1/3}CO_{1/3})O₂, Li(Ni_{0.6}Mn_{0.2}Co_{0.2})O₂, Li(Ni_{0.5}Mn_{0.3}Co_{0.2})O₂, Li(Ni_{0.7}Mn_{0.15}Co_{0.15})O₂, Li(Ni_{0.8}Mn_{0.1}Co_{0.1})O₂, *etc*.), a lithium nickel cobalt aluminum oxide (e.g., Li(Ni_{0.8}Co_{0.15}Al_{0.05})O₂, *etc.*)*,* a lithium nickel manganese cobalt aluminum oxide (e.g., Li(Ni_{0.86}Co_{0.05}Mn_{0.07}Al_{0.02})O₂), or the like, and any one thereof or a mixture of two or more thereof may be used.

Among these, in terms of the most improvement of the capacity characteristics of the battery, the positive electrode active material having a nickel content of 80 atm% or more may be used. For example, the lithium transition metal oxide may include one represented by Formula 2 below:

[Formula 2] LiₓNiₐCo_{b}M¹_{c}M²_{d}O₂

In Formula 2 above, M¹ above may be at least one selected from among Mn and Al, and preferably, Mn or a combination of Mn and Al.

M² may be at least one selected from the group consisting of Zr, B, W, Mg, Ce, Hf, Ta, La, Ti, Sr, Ba, F, P, and S.

x above represents an atomic fraction of the lithium in the lithium transition metal oxide, wherein x may satisfy 0.90≤x≤1.1, preferably 0.95≤x≤1.08, and more preferably 1.0≤x≤1.08.

a above represents an atomic fraction of nickel among the metallic elements other than the lithium in the lithium transition metal oxide, and may satisfy 0.80≤a<1.0, preferably 0.80≤a≤0.95, and more preferably 0.80≤a≤0.90. When the nickel content satisfies the above range, high capacity characteristics may be achieved.

b above represents an atomic fraction of cobalt among metallic elements other than the lithium in the lithium transition metal oxide, and may satisfy 0<b<0.2, 0<b≤0.15, or 0.01≤b≤0.10.

c above represents an atomic fraction of M¹ among the metallic elements other than the lithium in the lithium transition metal oxide, and may satisfy 0<c<0.2, 0<c≤0.15, or 0.01≤c≤0.10.

d above represents an atomic fraction of M² among the metallic elements other than the lithium in the lithium transition metal oxide, and may satisfy 0≤d≤0.1, or 0≤d≤0.05.

The positive electrode active material may be included in an amount of 60 wt% to 99 wt%, preferably 70 wt% to 99 wt%, and more preferably 80 wt% to 98 wt% based on the total weight of solids excluding the solvent in the positive electrode material mixture slurry.

The binder is a component that assists in the binding between the active material and the conductive agent and in the binding with the current collector.

Examples of the binder may include polyvinylidene fluoride, polyvinyl alcohol, carboxymethylcellulose (CMC), starch, hydroxypropylcellulose, regenerated cellulose, polyvinylpyrrolidone, polytetrafluoroethylene, polyethylene (PE), polypropylene, an ethylene-propylene-diene monomer, a sulfonated ethylene-propylene-diene monomer, a styrene-butadiene rubber, a fluoro rubber, various copolymers, and the like.

The binder may be commonly included in an amount of 1 wt% to 20 wt%, preferably 1 wt% to 15 wt%, and more preferably 1 wt% to 10 wt% based on the total weight of solid in excluding the solvent in the positive electrode material mixture slurry.

The conductive agent is a component for further improving the conductivity of the positive electrode active material, and may be added in an amount of 1 wt% to 20 wt% based on the total weight of the solid content in the positive electrode material mixture slurry. The conductive agent is not particularly limited as long as it has conductivity without causing adverse chemical changes in the battery, and, for example, a conductive material, such as: carbon powder such as carbon black, acetylene black, Ketjen black, channel black, furnace black, lamp black, or thermal black; graphite powder such as natural graphite with a well-developed crystal structure, artificial graphite, or graphite; conductive fibers such as carbon fibers or metal fibers; conductive powder such as fluorocarbon powder, aluminum powder, and nickel powder; conductive whiskers such as zinc oxide whiskers and potassium titanate whiskers; conductive metal oxide such as titanium oxide; or polyphenylene derivatives, may be used.

The conductive agent may be commonly included in an amount of 1 wt% to 20 wt%, preferably 1 wt% to 15 wt%, and more preferably 1 wt% to 10 wt% based on the total weight of solids excluding the solvent in the positive electrode material mixture slurry.

The solvent may include an organic solvent, such as N-methyl-2-pyrrolidone (NMP), and may be used in an amount such that desirable viscosity is obtained when the positive electrode active material as well as selectively the binder and the conductive agent are included. For example, the solvent may be included in an amount such that a concentration of a solid content including the positive electrode active material as well as selectively the binder and the conductive agent is in a range of 50 wt% to 95 wt%, preferably 70 wt% to 95 wt%, and more preferably 70 wt% to 90 wt%.

### (2) Negative Electrode

The negative electrode, for example, may be prepared by coating a negative electrode collector with a negative electrode material mixture slurry including a negative electrode active material, a binder, a conductive agent, and a solvent, or a graphite electrode formed of carbon (C) or a metal itself may be used as the negative electrode.

For example, in a case in which the negative electrode is prepared by coating the negative electrode collector with the negative electrode material mixture slurry, the negative electrode collector generally has a thickness of 3 µm to 500 µm. The negative electrode collector is not particularly limited so long as it has high conductivity without causing adverse chemical changes in the battery, and, for example, copper, stainless steel, aluminum, nickel, titanium, fired carbon, copper or stainless steel that is surface-treated with one of carbon, nickel, titanium, silver, or the like, an aluminum-cadmium alloy, or the like may be used. Also, similar to the positive electrode collector, the negative electrode collector may have fine surface roughness to improve bonding strength with the negative electrode active material, and the negative electrode collector may be used in various shapes such as a film, a sheet, a foil, a net, a porous body, a foam body, a non-woven fabric body, and the like.

Furthermore, the negative electrode active material may include at least one selected from the group consisting of lithium metal, a carbon material capable of reversibly intercalating/deintercalating lithium ions, metal or an alloy of lithium and the metal, a metal composite oxide, a material which may be doped and undoped with lithium, and a transition metal oxide.

As the carbon material capable of reversibly intercalating/deintercalating lithium ions, a carbon-based negative electrode active material generally used in a lithium ion secondary battery may be used without particular limitation, and, as a typical example, crystalline carbon, amorphous carbon, or both thereof may be used. Examples of the crystalline carbon may be graphite such as irregular, planar, flaky, spherical, or fibrous natural graphite or artificial graphite, and examples of the amorphous carbon may be soft carbon (low-temperature sintered carbon) or hard carbon, mesophase pitch carbide, and fired cokes.

As the metal or the alloy of lithium and the metal, a metal selected from the group consisting of Cu, Ni, Na, K, Rb, Cs, Fr, Be, Mg, Ca, Sr, Si, Sb, Pb, In, Zn, Ba, Ra, Ge, Al, and Sn, or an alloy of lithium and the metal may be used.

One selected from the group consisting of PbO, PbO₂, Pb₂O₃, Pb₃O₄, Sb₂O₃, Sb₂O₄, Sb₂O₅, GeO, GeO₂, Bi₂O₃, Bi₂O₄, Bi₂O₅, LiₓFe₂O₃ (0≤x≤1), LiₓWO₂ (0≤x≤1), and SnₓMe₁₋ₓMe'_{y}O_{z} (Me: Mn, Fe, Pb, Ge; Me': Al, B, P, Si, Groups I, II and III elements of the periodic table, or halogen; O<x≤1; 1≤y≤3; 1≤z≤8) may be used as the metal composite oxide.

The material, which may be doped and undoped with lithium, may include Si, SiOₓ (0<x≤2), a Si-Y alloy (where Y is an element selected from the group consisting of alkali metal, alkaline earth metal, a Group 13 element, a Group 14 element, transition metal, a rare earth element, and a combination thereof, and is not Si), Sn, SnO₂, and Sn-Y (where Y is an element selected from the group consisting of alkali metal, alkaline earth metal, a Group 13 element, a Group 14 element, transition metal, a rare earth element, and a combination thereof, and is not Sn), and a mixture of SiO₂ and at least one thereof may also be used. The element Y may be selected from the group consisting of Mg, Ca, Sr, Ba, Ra, Sc, Y, Ti, Zr, Hf, Rf, V, Nb, Ta, Db, Cr, Mo, W, Sg, Tc, Re, Bh, Fe, Pb, Ru, Os, Hs, Rh, Ir, Pd, Pt, Cu, Ag, Au, Zn, Cd, B, Al, Ga, Sn, In, Ge, P, As, Sb, Bi, S, Se, Te, Po, and a combination thereof.

The transition metal oxide may include lithium-containing titanium composite oxide (LTO), vanadium oxide, and lithium vanadium oxide.

Among these, the negative electrode active material may be a mixture of graphite and SiOₓ (0<x≤2). In terms of increasing the capacity of the lithium secondary battery, the graphite and SiOₓ (0<x≤2) may be included in a weight ratio of 97:3 to 70:30.

The negative electrode active material may be included in an amount of 60 wt% to 99 wt%, preferably 70 wt% to 99 wt%, and more preferably 80 wt% to 98 wt% based on the total weight of the solid content in the negative electrode material mixture slurry.

The binder is a component that assists in the binding between the conductive agent, the active material, and the current collector. Examples of the binder may include polyvinylidene fluoride (PVDF), polyvinyl alcohol, carboxymethylcellulose (CMC), starch, hydroxypropylcellulose, regenerated cellulose, polyvinylpyrrolidone, polytetrafluoroethylene, polyethylene, polypropylene, an ethylene-propylene-diene monomer, a sulfonated ethylene-propylene-diene monomer, a styrene-butadiene rubber, a fluoro rubber, and various copolymers thereof.

The binder may be commonly included in an amount of 1 wt% to 20 wt%, preferably 1 wt% to 15 wt%, and more preferably 1 wt% to 10 wt% based on the total weight of solids excluding the solvent in the negative electrode material mixture slurry.

The conductive agent is a component for further improving the conductivity of the negative electrode active material, and may be added in an amount of 1 wt% to 20 wt% based on the total weight of the solid content in the negative electrode material mixture slurry. The conductive agent is not particularly limited as long as it has conductivity without causing adverse chemical changes in the battery, and, for example, a conductive material, such as: carbon powder such as carbon black, acetylene black, Ketjen black, channel black, furnace black, lamp black, or thermal black; graphite powder such as natural graphite with a well-developed crystal structure, artificial graphite, or graphite; conductive fibers such as carbon fibers or metal fibers; conductive powder such as fluorocarbon powder, aluminum powder, and nickel powder; conductive whiskers such as zinc oxide whiskers and potassium titanate whiskers; conductive metal oxide such as titanium oxide; or polyphenylene derivatives, may be used.

The conductive agent may be included in an amount of 1 wt% to 20 wt%, preferably 1 wt% to 15 wt%, and more preferably 1 wt% to 10 wt% based on the total weight of solids excluding the solvent in the negative electrode material mixture slurry.

The solvent may include water or an organic solvent, such as N-methyl-2-pyrrolidone (NMP), and may be used in an amount such that desirable viscosity is obtained when the negative electrode active material as well as optionally the binder and the conductive agent is included. For example, the solvent may be included in an amount such that a concentration of a solid content including the negative electrode active material as well as optionally the binder and the conductive agent is in a range of 50 wt% to 95 wt%, for example, 70 wt% to 90 wt%.

In a case in which the metal itself is used as the negative electrode, the negative electrode may be prepared by a method of physically bonding, rolling, or depositing a metal on a metal thin film itself or the negative electrode collector. The depositing method may use an electrical deposition method or chemical deposition method of metal.

For example, the metal bonded/rolled/deposited on the metal thin film itself or the negative electrode collector may include one metal selected from the group consisting of lithium (Li), nickel (Ni), tin (Sn), copper (Cu), and indium (In) or an alloy of two metals thereof.

### (3) Separator

Also, a typical porous polymer film used as a typical separator, for example, a porous polymer film prepared from a polyolefin-based polymer, such as an ethylene homopolymer, a propylene homopolymer, an ethylene-butene copolymer, an ethylene-hexene copolymer, and an ethylene-methacrylate copolymer, may be used alone or in a lamination therewith as the separator. Also, a typical porous nonwoven fabric, for example, a nonwoven fabric formed of high melting point glass fibers or polyethylene terephthalate fibers may be used, but the present invention is not limited thereto. Furthermore, a coated separator including a ceramic component or a polymer material may be used to secure heat resistance or mechanical strength, and the separator having a single layer or multilayer structure may be optionally used.

A shape of the lithium secondary battery of the present invention is not particularly limited, but a cylindrical type using a can, a prismatic type, a pouch type, or a coin type may be used.

Hereinafter, the present invention will be described in more detail with reference to specific examples. However, the following examples are merely presented to exemplify the present invention, and it will be apparent to those skilled in the art that various modifications and alterations are possible within the scope of the present invention as set out in the appended claims.

### Examples

### Example 1

### (Preparation of Non-aqueous Electrolyte)

LiPF₆ (1.0 M), vinylenecarbonate (VC, 0.5 wt%), 1,3-propane sultone (PS, 0.5 wt%), and ethylene sulfate (Esa, 1.0 wt%) were dissolved in an organic solvent (ethylene carbonate (Ec):ehtylmethyl carbonate (EMC) = a volume ratio of 30:70) to prepare a non-aqueous solvent, 0.3 g of the compound of Formula 2-1 below was added to 99.7 g of the non-aqueous solvent to prepare a non-aqueous electrolyte.

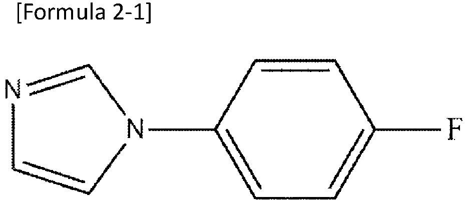

### (Manufacture of Lithium Secondary Battery)

A positive electrode active material (LiNi_{0.90}Co_{0.03}Mn_{0.06}Al_{0.01}O₂), a conductive agent (carbon black), and a binder (polyvinylidene fluoride) were added in a weight ratio of 97.6:0.8:1.6 to N-methyl-2-pyrrolidone (NMP), which was a solvent, to prepare a positive electrode slurry (solid content 60 wt%). The positive electrode slurry was applied on one surface of a positive electrode collector (Al thin film) having a thickness of 13.5 µm, dried, and roll-pressed to prepare a positive electrode.

A negative electrode active material (graphite:SiO = a weight ratio of 90:10), a conductive agent (carbon black), and a binder (SBR-CMC) were added in a weight ratio of 95.6:1.0:3.4 to N-methyl-2-pyrrolidone (NMP), which was a solvent, to prepare a negative electrode slurry (solid content 60 wt%). The negative electrode slurry was applied on one surface of a negative electrode current collector (Cu thin film) having a thickness of 6 µm, dried, and roll-pressed to prepare a negative electrode.

In a dry room, a porous separator was interposed between the positive electrode and the negative electrode prepared above, and then the prepared non-aqueous electrolyte was injected thereto to manufacture a secondary battery.

### Example 2

A secondary battery was manufactured in the same manner as in Example 1 except that 0.3 g of the compound of Formula 2-4 above was introduced to 99.7 g of the non-aqueous solvent prepared in Example 1 above to prepare a non-aqueous electrolyte.

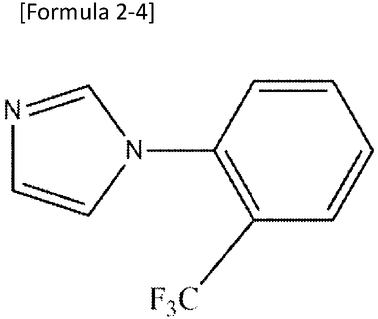

### Comparative Example 1

A secondary battery was manufactured in the same manner as in Example 1 except that 100 g of the non-aqueous solvent prepared in Example 1 was used to prepare a non-aqueous electrolyte.

### Comparative Example 2

A secondary battery was manufactured in the same manner as in Example 1 except that 0.3 g of N-phneylimidazole was introduced to 99.7 g of the non-aqueous solvent prepared in Example 1 to prepare a non-aqueous electrolyte.

### Experimental Example - Evaluation of High-temperature Storage Characteristics

For each of the secondary batteries manufactured in Examples 1 and 2 and Comparative Examples 1 and 2, cycle characteristics were evaluated.

Specifically, each of the batteries manufactured in Examples 1 and 2 and Comparative Examples 1 and 2 above was activated at a constant current (CC) of 0.1 C, and degasing was performed.

Subsequently, each secondary battery was charged at a CC of 0.33 C to 4.20 V under a constant current-constant voltage (CC-CV) condition at 25 °C, and then stored at 60 °C for 4 weeks. After 4 weeks of high-temperature storage, the amount of gas generated in the lithium secondary battery was measured. In addition, capacity retention after high-temperature storage was measured based on the capacity measured before high-temperature storage. The results are listed in Table 1 below:

**[Table 1]**

| | Amount of gas generated (mL) | Capacity retention (%) |
|---|---|---|
| Example 1 | 0.132 | 93.04 |
| Example 2 | 0.145 | 92.94 |
| Comparative Example 1 | 0.176 | 92.62 |
| Comparative Example 2 | 0.167 | 92.83 |

As shown in Table 1, it was confirmed that Examples 1 and 2 in which the additive for a non-aqueous electrolyte of the present invention was used had reduced amount of gas generated at high temperatures and excellent capacity retention effect as compared with Comparative Example 1 in which the additive was not used.

In addition, it was confirmed that Examples 1 and 2 in which the additive for a non-aqueous electrolyte of the present invention was used had reduced amount of gas generated and excellent capacity retention effect as compared with Comparative Example 2 in which N-phenylimidazole was used as the additive. It is thought that this is because when F is substituted at the benzene ring like the compound of Formula 1, F is easily deintercalated and thus a LiF film is easily formed.

## Claims

1. A non-aqueous electrolyte comprising:
a lithium salt including LiPF₆;
an organic solvent; and
an additive for a non-aqueous electrolyte represented by Formula 1 below: 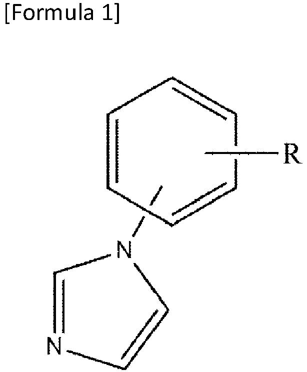
wherein in Formula 1 above, R is any one selected from the group consisting of a halogen element and a haloalkyl group having 1 to 10 carbon atoms, and
wherein the additive for a non-aqueous electrolyte is contained in an amount of 0.1 parts by weight to 5 parts by weight based on 100 parts by weight of the non-aqueous electrolyte.

2. The non-aqueous electrolyte of claim 1, wherein R above is any one selected from the group consisting of F, Cl, and a haloalkyl group having 1 to 5 carbon atoms.

3. The non-aqueous electrolyte of claim 1, wherein R above is any one selected from the group consisting of F, CF₃, and CF₂CF₃.

4. The non-aqueous electrolyte of claim 1, wherein the additive for a non-aqueous electrolyte represented by Formula 1 above is any one selected from the group consisting of Formula 1-1 and Formula 1-2 below: 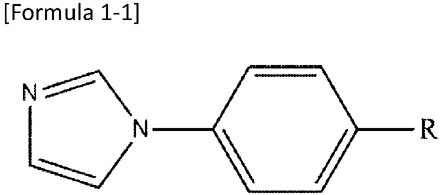 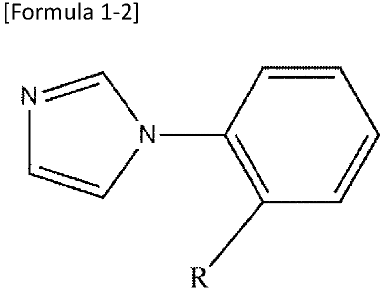 wherein in Formulae 1-1 and 1-2 above, R is any one selected from the group consisting of a halogen element and a haloalkyl group having 1 to 10 carbon atoms.

5. The non-aqueous electrolyte of claim 1, wherein the additive for a non-aqueous electrolyte represented by Formula 1 above is any one from among compounds represented by Formula 2-1 to Formula 2-4 below:

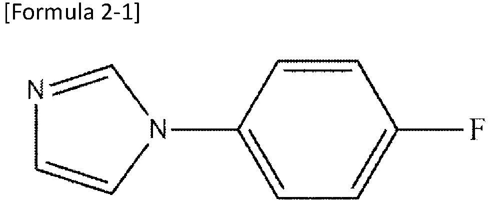

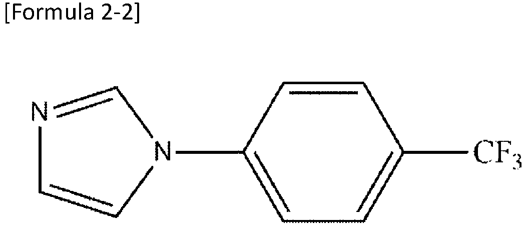

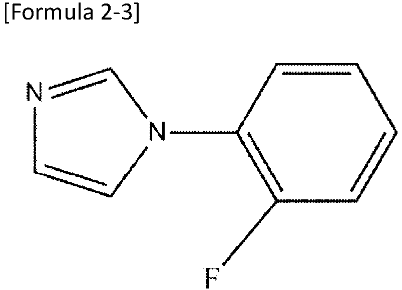

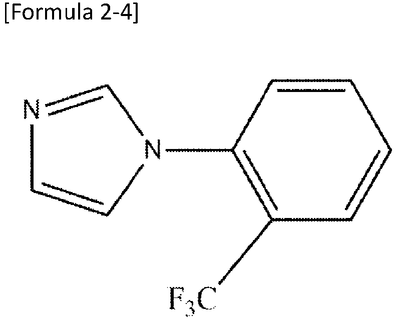

6. The non-aqueous electrolyte of claim 1, wherein the non-aqueous electrolyte further comprises at least one lithium salt selected from among LiCl, LiBr, Lil, LiBF₄, LiClO₄, LiB₁₀Cl₁₀, LiAlCl₄, LiAlO₂, LiSO₃CH₃, LiSO₃CF₃, LiCO₂CH₃, LiCO₂CF₃, LiAsF₆, LiSbF₆, LiN(SO₂F)₂ LiN(SO₂CF₂CF₃)₂, and LiN(SO₂CF₃)₂.

7. The non-aqueous electrolyte of claim 1, wherein the lithium salt is contained in a concentration of 0.5 M to 4.0 M.

8. The non-aqueous electrolyte of claim 1, wherein the organic solvent comprises at least one organic solvent selected from the group consisting of a cyclic carbonate-based organic solvent, a linear carbonate-based organic solvent, a linear ester-based organic solvent, and a cyclic ester-based organic solvent.

9. The non-aqueous electrolyte of claim 1, further comprising, as an additive, at least one compound selected from the group consisting of a cyclic carbonate-based compound, a halogen-substituted carbonate-based compound, a sultone-based compound, a sulfate-based compound, a phosphate-based compound, a borate-based compound, a nitrile-based compound, a benzene-based compound, an amine-based compound, a silane-based compound, and a lithium salt-based compound.

10. A lithium secondary battery comprising the non-aqueous electrolyte of any one of claims 1 to 9.

## Patentansprüche

1. Nichtwässriger Elektrolyt, umfassend:
ein Lithiumsalz, das LiPF₆ enthält;
ein organisches Lösungsmittel; und
ein Additiv für einen nichtwässrigen Elektrolyten, dargestellt durch nachfolgende Formel 1: 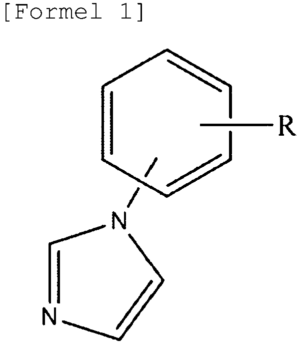
worin, in obiger Formel 1, R eines ist, ausgewählt aus der Gruppe, bestehend aus einem Halogenelement und einer Halogenalkylgruppe mit 1 bis 10 Kohlenstoffatomen, und
worin das Additiv für einen nichtwässrigen Elektrolyten in einer Menge von 0,1 Gewichtsteilen bis 5 Gewichtsteilen, basierend auf 100 Gewichtsteilen des nichtwässrigen Elektrolyten, enthalten ist.

2. Nichtwässriger Elektrolyt gemäß Anspruch 1, worin R oben eines ist, ausgewählt aus der Gruppe, bestehend aus F, Cl und einer Halogenalkylgruppe mit 1 bis 5 Kohlenstoffatomen.

3. Nichtwässriger Elektrolyt gemäß Anspruch 1, worin R oben eines ist, ausgewählt aus der Gruppe, bestehend aus F, CF₃ und CF₂CF₃.

4. Nichtwässriger Elektrolyt gemäß Anspruch 1, worin das durch obige Formel 1 dargestellte Additiv für einen nichtwässrigen Elektrolyten eines ist, ausgewählt aus der Gruppe, bestehend aus nachfolgender Formel 1-1 und Formel 1-2: 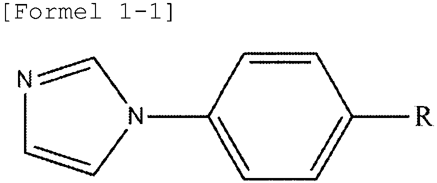 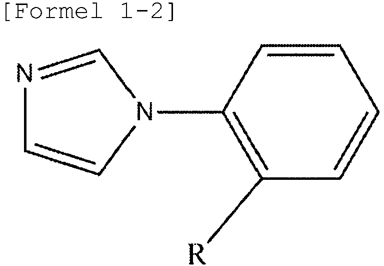 worin, in den obigen Formeln 1-1 und 1-2, R eines ist, ausgewählt aus der Gruppe, bestehend aus einem Halogenelement und einer Halogenalkylgruppe mit 1 bis 10 Kohlenstoffatomen.

5. Nichtwässriger Elektrolyt gemäß Anspruch 1, worin das durch obige Formel 1 dargestellte Additiv für einen nichtwässrigen Elektrolyten eine der durch die nachstehenden Formeln 2-1 bis 2-4 dargestellten Verbindungen ist:

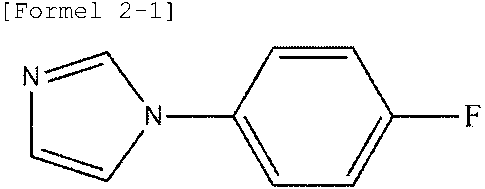

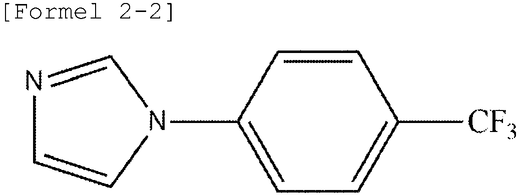

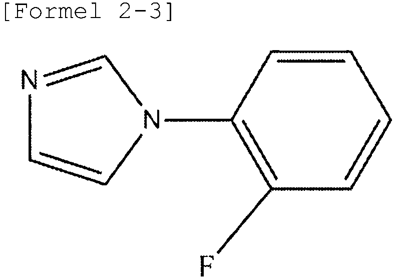

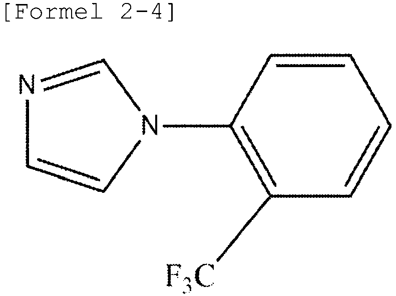

6. Nichtwässriger Elektrolyt gemäß Anspruch 1, worin der nichtwässrige Elektrolyt ferner mindestens ein Lithiumsalz umfasst, ausgewählt aus LiCl, LiBr, LiI, LiBF₄, LiClO₄, LiB₁₀Cl₁₀, LiAlCl₄, LiAlO₂, LiSO₃CH₃, LiSO₃CF₃, LiCO₂CH₃, LiCO₂CF₃, LiAsF₆, LiSbF₆, LiN(SO₂F)₂ LiN(SO₂CF₂CF₃)₂ und LiN(SO₂CF₃)₂.

7. Nichtwässriger Elektrolyt gemäß Anspruch 1, worin das Lithiumsalz in einer Konzentration von 0,5 M bis 4,0 M enthalten ist.

8. Nichtwässriger Elektrolyt gemäß Anspruch 1, worin das organische Lösungsmittel mindestens ein organisches Lösungsmittel umfasst, ausgewählt aus der Gruppe, bestehend aus einem organischen Lösungsmittel auf Basis eines cyclischen Carbonats, einem organischen Lösungsmittel auf Basis eines linearen Carbonats, einem organischen Lösungsmittel auf Basis eines linearen Esters und einem organischen Lösungsmittel auf Basis eines cyclischen Esters.

9. Nichtwässriger Elektrolyt gemäß Anspruch 1, ferner umfassend, als Additiv, mindestens eine Verbindung, ausgewählt aus der Gruppe, bestehend aus einer Verbindung auf Basis eines cyclischen Carbonats, einer Verbindung auf Basis eines halogensubstituierten Carbonats, einer Verbindung auf Basis von Sulton, einer Verbindung auf Basis von Sulfat, einer Verbindung auf Basis von Phosphat, einer Verbindung auf Basis von Borat, einer Verbindung auf Basis von Nitril, einer Verbindung auf Basis von Benzol, einer Verbindung auf Basis von Amin, einer Verbindung auf Basis von Silan und einer Verbindung auf Basis eines Lithiumsalzes.

10. Lithiumsekundärbatterie, umfassend den nichtwässrigen Elektrolyten gemäß ein der Ansprüche 1 bis 9.

## Revendications

1. Électrolyte non aqueux comprenant :
un sel de lithium incluant du LiPF₆ ;
un solvant organique ; et
un additif pour électrolyte non aqueux représenté par la Formule 1 ci-dessous : 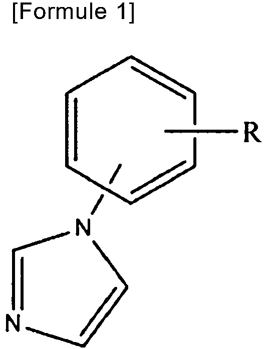
dans lequel, dans la Formule 1 ci-dessus, R est l'un quelconque sélectionné dans le groupe consistant en un élément halogène et un groupe haloalkyle présentant 1 à 10 atomes de carbone, et
dans lequel l'additif pour électrolyte non aqueux est contenu dans une quantité de 0,1 partie en poids à 5 parties en poids sur la base de 100 parties en poids de l'électrolyte non aqueux.

2. Électrolyte non aqueux selon la revendication 1, dans lequel R ci-dessus est l'un quelconque sélectionné dans le groupe consistant en F, CI, et un groupe haloalkyle présentant 1 à 5 atomes de carbone.

3. Électrolyte non aqueux selon la revendication 1, dans lequel R ci-dessus est l'un quelconque sélectionné dans le groupe consistant en F, CF₃, et CF₂CF₃.

4. Électrolyte non aqueux selon la revendication 1, dans lequel l'additif pour électrolyte non aqueux représenté par la Formule 1 ci-dessus est l'un quelconque sélectionné dans le groupe consistant en la Formule 1-1 et la Formule 1-2 ci-dessous : 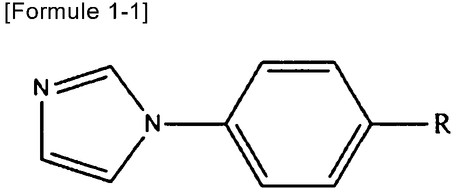 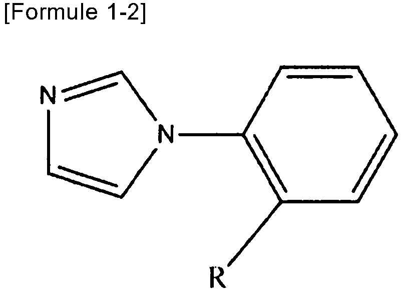 dans lequel, dans les Formules 1-1 et 1-2 ci-dessus, R est l'un quelconque sélectionné dans le groupe consistant en un élément halogène et un groupe haloalkyle présentant 1 à 10 atomes de carbone.

5. Électrolyte non aqueux selon la revendication 1, dans lequel l'additif pour électrolyte non aqueux représenté par la Formule 1 ci-dessus est l'un quelconque sélectionné parmi les composés représentés par les Formule 2-1 à Formule 2-4 ci-dessous :

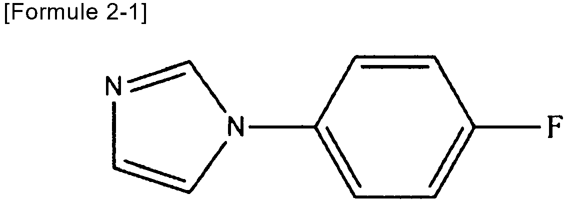

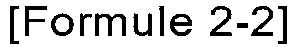

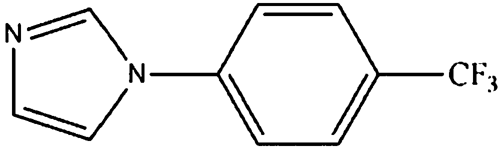

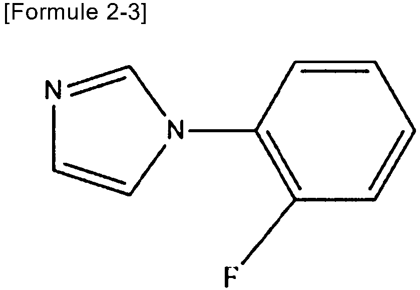

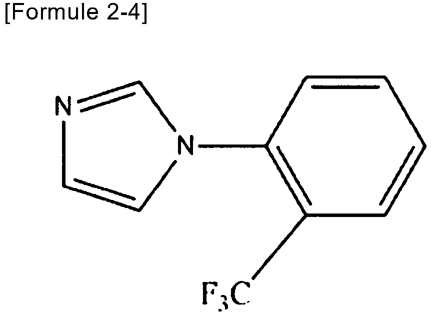

6. Électrolyte non aqueux selon la revendication 1, dans lequel l'électrolyte non aqueux comprend en outre au moins un sel de lithium sélectionné parmi LiCl, LiBr, Lil, LiBF₄, LiClO₄, LiB₁₀Cl₁₀, LiAlCl₄, LiAlO₂, LiSO₃CH₃, LiSO₃CF₃, LiCO₂CH₃, LiCO₂CF₃, LiAsF₆, LiSbF₆, LiN(SO₂F)₂, LiN(SO₂CF₂CF₃)₂, et LiN(SO₂CF₃)₂.

7. Électrolyte non aqueux selon la revendication 1, dans lequel le sel de lithium est contenu dans une concentration de 0,5 M à 4,0 M.

8. Électrolyte non aqueux selon la revendication 1, dans lequel le solvant organique comprend au moins un solvant organique sélectionné dans le groupe consistant en un solvant organique à base de carbonate cyclique, un solvant organique à base de carbonate linéaire, un solvant organique à base d'ester linéaire, et un solvant organique à base d'ester cyclique.

9. Électrolyte non aqueux selon la revendication 1, comprenant en outre, en tant qu'additif, au moins un composé sélectionné dans le groupe consistant en un composé à base de carbonate cyclique, un composé à base de carbonate substitué par un halogène, un composé à base de sultone, un composé à base de sulfate, un composé à base de phosphate, un composé à base de borate, un composé à base de nitrile, un composé à base de benzène, un composé à base d'amine, un composé à base de silane, et un composé à base de sel de lithium.

10. Batterie secondaire au lithium comprenant l'électrolyte non aqueux selon l'une quelconque des revendications 1 à 9.
